(19)

Europäisches
Patentamt
European
Patent Office
Office européen
des brevets

(11) **EP 3 864 969 A1**

(12) **EUROPEAN PATENT APPLICATION**
published in accordance with Art. 153(4) EPC

(43) Date of publication:
**18.08.2021 Bulletin 2021/33**

(21) Application number: **19872050.0**

(22) Date of filing: **09.10.2019**

(51) Int Cl.:
**A23L 19/00** *(2016.01)*        **A23L 33/00** *(2016.01)*

(86) International application number:
**PCT/CN2019/110142**

(87) International publication number:
**WO 2020/073922 (16.04.2020 Gazette 2020/16)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **10.10.2018 HK 18112945**

(71) Applicant: **Leung, So Ngor Sally
Hong Kong (CN)**

(72) Inventors:
• **ZHANG, Xiu Cheng
Hong Kong (CN)**
• **LEUNG, So Ngor Sally
Hong Kong (CN)**

(74) Representative: **Isern Patentes y Marcas S.L.
Avda. Diagonal, 463 Bis, 2°
08036 Barcelona (ES)**

(54) **HEALTH PRODUCT COMPOSITION FOR SUPPLEMENTING DEFICIENCY, PREPARATION METHOD THEREFOR AND APPLICATION THEREOF**

(57) The present invention relates to a health-care product composition for enriching asthenic disease, a preparation method therefor and use thereof. According to parts by mass, the raw materials required for preparing the composition comprise: 5 to 40 parts of dandelions, 10 to 120 parts of bulbous vegetables, 10 to 100 parts of condiment leafy vegetables, and 8 to 16 parts of fruits. The method for preparing the health-care product composition for enriching asthenic disease comprises the following steps: the raw materials such as dandelion, bulbous vegetables, and condiment leafy vegetables are pulverized and mixed to obtain a solid mixture; the solid mixture is leached with purified water and filtered to obtain a leaching juice; the leaching juice is concentrated to obtain a concentrated liquid; ethanol is added to the concentrated liquid to precipitate and remove impurities so as to obtain a refining solution; the refining solution is subjected to embedding treatment so as to obtain an extracting solution; the extracting solution is lyophilized to obtain a lyophilized powder of the health-care product composition for enriching asthenic disease. The health-care product composition for enriching asthenic disease prepared by the method contain no hormones, has no obvious toxic and side effects, and has the efficacy of enriching asthenic disease, tonifying kidney and strengthening yang.

| |
|---|
| Pulverizing and mixing the raw materials to obtain a solid mixture. — S110 |
| Adding purified water to the solid mixture for leaching treatment, and filtering to obtain a leaching juice. — S120 |
| Concentrating the leaching juice to obtain a concentrated liquid. — S130 |
| Refining the concentrated liquid by adding ethanol so as to obtain a refining solution. — S140 |
| Adding an embedding medium for embedding treatment so as to obtain an extracting solution. — S150 |
| Lyophilizing the extracting solution to obtain a lyophilized powder of the health-care product composition for enriching asthenic disease. — S160 |
| Adding adjuvant materials to the above-mentioned leaching juice or lyophilized powder; mixing and formulating to prepare a liquid beverage or a solid beverage. — S170 |

*Fig. 1*

**Description**

This application claims the priority of HEALTH-CARE PRODUCT COMPOSITION FOR ENRICHING ASTHENIC DISEASE, PREPARATION METHOD THEREFOR AND USE THEREOF with Hong Kong short-term patent application number 18112945.1 in the Intellectual Property Department of Hong Kong Special Administrative Region on October 10, 2018, and the disclosure is incorporated herein in its entirety by reference.

**Technical Field**

The present invention relates to the field of health-care products, in particular to a health-care product composition for enriching asthenic disease, a preparation method therefor and use thereof.

**Background Art**

With the accelerating pace of life, work and life pressures continue to increase, and the number of people in sub-health status is also increasing. Especially for male, they often have symptoms such as lack of physical strength, yin deficiency of liver and kidney and yang deficiency of kidney at middle age. This brings great mental stress to patients and even affects normal family life.

For this reason, patients often need to seek help with medicines. Although there are many kinds of medicines for tonifying kidney and strengthening yang currently on the market, most of them contain sex hormones, or have obvious side effects, and long-term usage will cause medicine dependence and even harm the health of the body.

**Summary of the invention**

On this basis, it is necessary to provide a health-care product composition for enriching asthenic disease which has no obvious toxic and side effects and has the efficacy of enriching asthenic disease, tonifying kidney and strengthening yang.

In addition, a method for preparing the health-care product composition for enriching asthenic disease and a use of the composition are further provided.

The present invention relates to a health-care product composition for enriching asthenic disease, and according to parts by mass, the raw materials for preparing the health-care product composition for enriching asthenic disease comprise:

5 to 40 parts of dandelions, 10 to 120 parts of bulbous vegetables, 10 to 100 parts of condiment leafy vegetables, and 8 to 16 parts of fruits;
wherein the bulbous vegetables are selected from at least one of scallion, onion and garlic; the condiment leafy vegetables are selected from at least one of Chinese chive, celery and coriander; the fruits are selected from at least one of grape, blueberry, blackcurrant, lemon, tangerine, orange, litchi, cherry, longan aril, strawberry, pineapple, coconut, pitaya, yacon, durian, noni fruit, *Solanum mammosum L.* and tomato.

The raw materials of the above-mentioned health-care product composition for enriching asthenic disease are all selected from common vegetables and fruits, contain no sex hormones and have no obvious toxic and side effects; Moreover, the inventors confirm through clinical trials that the above-mentioned health-care product composition for enriching asthenic disease prepared from the raw materials has the efficacy of enriching asthenic disease, tonifying kidney and strengthening yang.

In one of the examples, according to parts by mass, the raw materials for preparing the health-care product composition for enriching asthenic disease further comprise: 0 to 6 parts of edible seeds, which are selected from at least one of barbary wolfberry fruit, emblic, seabuckthorn fruit, oat, tartarian buckwheat, natto, mulberry fruit, walnut seed, sesame, tree peony seed, passion fruit, bitter apricot seed, peach seed, carthamus seed, perilla fruit, cushaw seed, bitter gourd seed, pine seed, moringa seed, seed of *Lepidium meyenii Walp.,* okra seed, hawthorn fruit, cacao bean and coffee bean.

In one of the examples, the raw materials are characterized in that according to parts by mass, the raw materials for preparing the health-care product composition for enriching asthenic disease further comprise 0 to 6 parts of jinhua fungus of tea seeds and 0 to 6 parts of pine pollens.

In one of the examples, according to parts by mass, the raw materials for preparing the health-care product composition for enriching asthenic disease further comprise: 0 to 6 parts of condiment materials, which are selected from at least one of ginger, cinnamon, fennel, star anise, pepper fruit, *Ligusticum chuanxiong Hort.,* pricklyash peel and clove.

The present invention relates to the use of the health-care product composition for enriching asthenic disease of any one of the above-mentioned examples for the preparation of a health-care medicine, a health-care beverage or chocolate.

In one of the examples, the method for preparing the above-mentioned health-care product composition for enriching asthenic disease comprises the following steps: the raw materials are pulverized, mixed, and then subjected to leaching treatment in purified water at 70°C to 80°C so as to obtain a leaching juice.

In one of the examples, after the step of the leaching treatment, the method further comprises the following step:

the leaching juice is concentrated to obtain a concentrated liquid having a relative density of 1.18 to 1.20; and an embedding medium having a volume of 0.3% to 6% of the concentrated liquid volume is added for embedding treatment so as to obtain an extracting solution.

In one of the examples, when concentration is performed, the reverse osmotic pressure is 30 kg/cm$^2$ to 150 kg/cm$^2$, and the embedding medium is β-cyclodextrin or porous starch.

In one of the examples, after the step of concentration treatment and before the step of embedding treatment, the method further comprises the following steps: ethanol solution with an volume 3 times the volume of the concentrated liquid is added to precipitate and remove the impurities so as to obtain a refining solution, wherein the volume percentage content of ethanol in the ethanol solution is higher than 70%.

In one of the examples, after the step of embedding treatment, the method further comprises the following step: the extracting solution is lyophilized at a temperature of -70°C to -35°C so as to prepare a lyophilized powder.

**Brief Description of the Drawings**

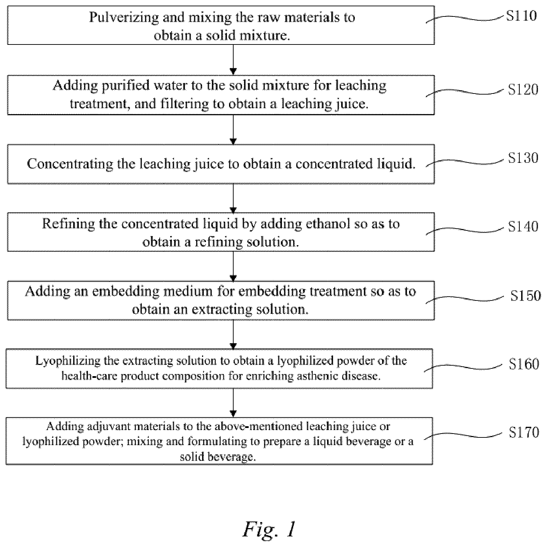

Figure 1 is a flow chart showing a method for preparing the health-care product according to an example.

**Detailed Description of Embodiments**

In order to facilitate the understanding of the present invention, the present invention will be described more comprehensively below with reference to relevant accompanying drawings. Preferred examples of the present invention are shown in the drawings. However, the present invention may be embodied in many different forms and is not limited to the examples described herein. Rather, these examples are provided so that the disclosure of the present invention will be more fully and comprehensively understood.

Unless otherwise defined, all technological and scientific terms used herein have the same meanings as commonly understood by those of ordinary skill in the art of the present invention. Herein, the terms used in the description of the present invention are merely for the purpose of describing specific embodiments, but are not intended to limit the present invention.

As shown in figure 1, a method for preparing the health-care product composition for enriching asthenic disease according to an embodiment comprises the following steps:

step S110: according to parts by mass, 5 to 40 parts of dandelions, 10 to 120 parts of bulbous vegetables, 10 to 100 parts of condiment leafy vegetables, 8 to 16 parts of fruits, 0 to 6 parts of edible seeds, 0 to 6 parts of jinhua fungus of tea seeds, 0 to 6 parts of pine pollens and 0 to 6 parts of condiment materials are pulverized and mixed to obtain a solid mixture.

Dandelion refers to the whole grass of the dandelion, the perennial herb plant in family *Asteraceae;* dandelion is sweet in taste, cold-natured, non-toxic and acts on liver and kidney channels; according to the classics of traditional Chinese medicine, it has functions of clearing heat and removing toxicity, and inducing diuresis for treating strangurtia.

according to "纲目 [Compendium of Materia Medica]": it has functions of "blackening the beard and hair, and strengthening muscles and bones"; according to "医林纂要 [YiLing CuanYao]": it has functions of "tonifying spleen and harmonizing stomach, nourishing yin and cooling blood, relieving rigidity of muscle and strengthening teeth, and promoting lactation and boosting essence, and also has effects of purging intense heat and quieting the spleen"; according to "本草求真 [Truth Seeking Herbal Foundation]": it can "act on stomatch meridian of foot-yangming and liver channel of jueyin, cool blood and relieve heat"; according to MOU Xiyong: it is "sweet in taste and neutral-natured, and can nourish liver and kidney".

The bulbous vegetables are selected from at least one of scallion, onion and garlic. scallion is acrid in taste, mild-natured, can act on channels of lung, stomach and liver, and has functions of removing cold-evil by sweating, activating yang and removing toxicity; it also has functions of easing joint movement, activating yang qi, dispersing wind pathogen, and can act on the surface and in the interior; scallion actually has functions of warming kidney, improving eyesight and curing impotence; scallion is uniquely spicy and pungent; the malic acid and phosphate sugar contained in the scallion can excite nerves, and improve and promote blood circulation. Garlic refers to the herb plant with scale-like bulbel of genus *Allium* in family *Liliaceae;* garlic is warm-natured, tastes spicy, and has functions of descending qi, eliminating wind, alleviating pain, and removing bad qi; garlic contains iron, magnesium, strontium and selenium, and further contains 17 kinds of amino acids with the highest arginine content, accounting for 20.4% of the total amino acids; garlic also contains allicin, a volatile sulfide formed by alliin by the action of alliinase; according to research, garlic has the physiological efficacy of lowering blood pressure, anti-platelet aggregation, anti-arteriosclerosis and cancer prevention; the phytoncide in garlic has a strong bactericidal effect.

It should be noted that the above-mentioned scallions and onions are not limited to specific varieties, for example, they may also be Chinese onions, shallots, *Allium victorialis L., Veratrum nigrum L., Allium ascalonicum,* etc. When referring to scallions, it is also not limited to specific edible parts, which, for example, may be leaves, stalks, flowers, seeds, etc. of the scallions. Similarly, the above-mentioned garlics are not limited to specific varieties, for example, they may be *Allium sativum,* black garlic, etc.; When referring to garlics, it is also not limited to specific edible parts, which, for example, may be stalks, bolts, shoots, etc. of the garlics.

The condiment leafy vegetables are selected from at least one of Chinese chive, celery and coriander. Chinese chive is acrid in taste, mild-natured, and can act on channels of lung, stomach and liver; according to "本草拾遗 [Gleaning Herb]": it has functions of "warming the middle warmer, descending qi, tonifying deficiency, and harmonizing zang-fu viscera"; according to "日华子本草 [Rihuazi Bencao]": it has functions of "preventing seminal emission and warming waist and knee"; according to "中药大辞典 [The Dictionary of Chinese Herbal Medicine]": it has functions of "warming the middle warmer, promoting the circulation of qi, dissipating the blood, and removing toxicity", and also has functions of "treating yang deficiency and kidney cold, depressed yang, or cold and pain in the waist and knees, spermatorrhea and nocturnal emission"; Chinese chive has high content of protein, fat and carbohydrate, is rich in vitamins, and abundant in minerals such as calcium, phosphorus and iron; it also contains volatile essential oils, sulfides and other ingredients, and has effects such as exciting nerves; Chinese chive further contains alkaloids and saponins, and has functions of warmly invigorating liver and kidney, controlling nocturnal emission and strengthening yang. Celery refers to a biennial herb plant that forms a fat and tender petiole in genus *Umbelliferae;* it can be divided into Chinese celery and western celery; celery has the effect of calming the liver for clearing heat; celery contains vitamin A, vitamin B1, vitamin B2, vitamin C, calcium, iron, phosphorus and other nutrients; celery contains more iron and is a good therapeutic supplement for iron deficiency. celery contains apigenin, bergamot lactone, volatile oil, butylphthalide and other ingredients; celery also contains a substance that relaxes smooth muscle, which has the effects of blood pressure reduction and brain strengthening. Coriander is an annual herb plant, leaves and tender stems of which are edible, in genus *Umbelliferae;* coriander has functions of promoting sweating and eruption, helping digestion for descending qi, and enlivening the spleen for regulating stomach; coriander contains calcium, phosphorus, iron, carotene, vitamin C, thiamine and riboflavin, and contains aromatic oils such as linalool, isocoumarin A and B, bergapten, etc., and coriander is the main source of aromatic substances.

It should be noted that the above-mentioned Chinese chive, celery, and coriander are not limited to specific varieties, and are also not limited to specific edible parts.

The fruits are selected from at least one of grape, blueberry, blackcurrant, lemon, tangerine, orange, litchi, cherry, longan aril, strawberry, pineapple, coconut, pitaya, yacon, durian, noni fruit, *Solanum mammosum L.* and tomato. Grapes are sweet and acrid in taste, neutral-natured, and can act on channels of lung, spleen and liver; according to "中药大辞典 [The Dictionary of Chinese Herbal Medicine]": grapes have functions of "benefiting qi and blood, strengthening muscles and bones, and inducing urination". according to "本经 [Sheng Nong's Herbal Classic]": grapes have functions of "treating arthritis with fixed pain caused by dampness, tonifying qi and increasing the strength, and enhancing memory ability", according to "滇南本草 [Materia Medica South Yunnan]": grapes have functions of "extraordinarily benefiting qi and blood, and stimulating the circulation of the blood and causing the muscles and joints to relax"; according to "随息居饮食谱 [With the Interest Rate Spectrum of Home Eating]": grapes have functions of "tonifying qi, nourishing kidney, nourishing liver yin, strengthening muscles and bones, quenching thirst, and tranquilizing fetus to prevent miscarriage"; according to "陆川本草 [Luchuan Herbs]": grapes have functions of "nourishing and strengthening body, enriching the blood, tonifying the heart, and promoting discharge of urine". According to "中药大辞典 [The Dictionary of Chinese Herbal Medicine]": lemon has functions of "promoting the production of body fluid, quenching thirst, relieving heatstroke, and tranquilizing fetus to prevent miscarriage"; according to "食物考 [Shiwu Kao]": lemon is "suitable for pregnant women and has functions of tranquilizing fetus to prevent miscarriage"; according to "纲目拾遗 [Supplement to Compendium of Materia Medica]": it has functions of "descending qi for regulating stomach"; lemon can improve muscle working ability, prevent fatigue and relieve existing fatigue. Blueberries have a high content of pectin, are rich in vitamin C, and contains trace elements of manganese; blueberry is an anthocyanin-rich fruit, and ranks first in the antioxidant capacity of more than 40 kinds of fruits and vegetables; the flavin contained in the blueberries prevents brain cells from being destroyed by free radicals; eating blueberries can prevent cancer and slow down aging, improve the balance and coordination of the elderly, especially has the effect of preventing memory loss and motor nerve degeneration; in addition, blueberries have functions of protecting capillaries, are good for heart health, contain polyphenol substance, anthocyanins and other ingredients, and can enhance human immunity; studies have shown that anthocyanins, the active ingredient of blueberries, can stimulate the synthesis of nitric oxide through vascular endothelial cells, thereby improving the quality of erection in male. Blackcurrant, also known as dry grapes, purple plum, black fruit, etc.; the fruit of blackcurrant is rich in anthocyanins, phenolic substances, substances containing sugars, a variety of vitamins and organic acids, etc.; especially, the fruit of blackcurrant has a extremely high content of vitamin C, and contains phosphorus, magnesium, potassium and calcium, of which zinc content is high; the seed of blackcurrant has high content of linoleic acid, and has antihypertensive, hypolipidemic and anti-arteriosclerosis effects; and another r-linolenic acid contained therein, which is a unique ingredient that cannot be synthesized in human body, can have a effect against cardiovascular diseases; the fruit of blackcurrant has functions of nourishing kidney and replenishing qi and blood, has health efficacy comprising: preventing gout, anemia, edema, arthritis, rheumatism, and oral and throat disorders, and has a good effect on heart and cerebral vessels, high blood pressure, etc.; blackcurrant has a high medicinal value, which can enhance human immunity and have functions of anti-aging. Tangerine has functions of stimulating appetite and regulating vital energy, and contains calcium, phosphorus, iron, potassium, carotene, vitamin B1, vitamin B2, vitamin C, etc.; a variety of organic acids and vitamins contained in the tangerine can excite the heart and reduce the fragility of capillaries, which has health benefits for the elderly and cardiovascular patients. Orange has functions of regulating vital energy, helping digestion for stimulating appetite, and reducing phlegm; orange is rich in a variety of organic acids and vitamins, which are very beneficial to the metabolism and heart in human body. In addition, the flavonoid substances contained in tangerine and orange can expand the coronary arteries and increase coronary blood flow, and are important substances for maintaining heart health.

It should be noted that the above-mentioned various fruits are not limited to the form thereof, and for example, they may be in the form of fresh fruit, dried fruit or fruit juice.

The edible seeds are selected from at least one of barbary wolfberry fruit, emblic, seabuckthorn fruit, oat, tartarian buckwheat, natto, mulberry fruit, walnut seed, sesame, tree peony seed, passion fruit, bitter apricot seed, peach seed, carthamus seed, perilla fruit, cushaw seed, bitter gourd seed, pine seed, moringa seed, seed of *Lepidium meyenii Walp.,* okra seed, hawthorn fruit, cacao bean and coffee bean.

The condiment materials are selected from at least one of ginger, cinnamon, fennel, star anise, pepper fruit, *Ligusticum chuanxiong Hort.,* pricklyash peel and clove.

Step S120: 800 ml of purified water are added to the above-mentioned solid mixture; the mixture is subjected to leaching for 1 h to 2 h after the temperature raising to 70°C to 80°C, and filtered to obtain a leaching juice.

Step S130: the above-mentioned leaching juice is concentrated to obtain a concentrated liquid having a relative density of 1.18 to 1.20 under a pressure of a reverse osmotic pressure of 30 kg/cm$^2$ to 150 kg/cm$^2$.

Step S140: ethanol solution with an volume 3 times the volume of the concentrated liquid is added to the above-mentioned concentrated liquid to precipitate and remove impurities so as to obtain a refining solution,

wherein the volume percentage content of ethanol in the ethanol solution is higher than 70%.

Step S150: An embedding medium having a volume of 0.3% to 6% of the concentrated liquid volume is added to the above-mentioned refining solution, stirred and mixed for embedding treatment so as to obtain an extracting solution, wherein the embedding medium is selected from at least one of β-cyclodextrin and porous starch.

Step S160: the above-mentioned extracting solution is lyophilized at -70°C to -35°C so as to prepare a lyophilized powder of the health-care product composition for enriching asthenic disease.

Step S170: according to parts by mass, 0.01 to 5 parts of sweetener, 0.01 to 0.05 parts of table salt, 0.3 to 0.5 parts of sodium bicarbonate, and 0.1 to 0.3 parts of flavoring materials are added to the above-mentioned extracting solution or lyophilized powder so as to prepare a liquid beverage or a solid beverage,

wherein the sweetener is selected from sweeteners commonly used in the field of food processing, which, for example, is at least one of glucose, brown sugar, honey, white granulated sugar, rock sugar, fructose, egg white and sugar, stevioside, xylitol, cyclamate and acesulfame. Similarly, the flavoring materials are also selected from commonly used flavoring material products, which will not be described herein.

It should be noted that the extracting solution prepared in steps S110 to S150 is used for preparing a liquid beverage; step S140 can be omitted, and after this step is omitted, the concentrated liquid in step S130 is directly subjected to the embedding treatment in step S150. The lyophilized powder prepared in step S160 is used to prepare a solid beverage, that is, if there is no need to prepare a solid beverage, step S160 is not necessary. Step S170 is used for preparing the extracting solution in step S150 into a health-care liquid beverage product, or for preparing the lyophilized powder in step S160 into a health-care solid beverage product.

According to the method for preparing the above-mentioned health-care product composition for enriching asthenic disease, the above-mentioned various raw materials are pulverized and mixed in the above-mentioned ratio, and then treated according to the corresponding steps to prepare a liquid or solid health-care product composition for enriching asthenic disease. The above-mentioned health-care product composition for enriching asthenic disease is added with an appropriate amount of food additives, and the food processing technology can be used for preparing products such as health-care medicine, health-care beverage (including health-care vinegar, health-care wine, enzymes, etc.) or chocolate. The dosage form of the health-care medicine is a commonly-used dosage form such as an oral liquid, a capsule, a pill, a tablet, or a honey ointment.

The raw materials for preparing the health-care product composition for enriching asthenic disease by the above-mentioned preparation method are all selected from vegetables, fruits or medical and edible materials, contain no hormones and have no obvious toxic and side effects. In addition, it has been proved by clinical trials that the health-care product composition for enriching asthenic disease has the efficacy of enriching asthenic disease, tonifying kidney and strengthening yang.

The example part is as below:

Example 1

The preparation process of the health-care product composition for enriching asthenic disease of the present example is as follows:

(1) according to parts by mass, 25 parts of dandelions, 35 parts of scallions, 10 parts of Chinese chives, 8 parts of mixtures of grapes with blueberries and blackcurrants, 3 parts of jinhua fungus of tea seeds, 3 parts of pine pollens, 3 parts of mixtures of cushaw seeds with bitter gourd seeds and sesame seeds, and 3 parts of gingers are pulverized and mixed so as to obtain a solid mixture;

(2) 800 ml of purified water are added to the above-mentioned solid mixture; the mixture is subjected to leaching for 100 min after the temperature raising to 70°C, and filtered to obtain a leaching juice;

(3) the above-mentioned leaching juice is concentrated to obtain a concentrated liquid having a relative density of 1.18 under a pressure of a reverse osmotic pressure of 30 kg/cm$^2$;

(4) ethanol solution (70% by volume percentage content of ethanol) with an volume 3 times the volume of the concentrated liquid is added to the above-mentioned concentrated liquid to precipitate and remove impurities so as to obtain a refining solution;

(5) β-cyclodextrin having a volume of 0.3% of the concentrated liquid volume is added to the above-mentioned refining solution, stirred and mixed for embedding treatment so as to obtain an extracting solution;

(6) according to parts by mass, 3 parts of glucose and 0.01 parts of table salt are added to the above-mentioned extracting solution and the mixture is heated to 76.6°C for sterilization for 2 minutes so as to formulate and prepare a liquid beverage of the health-care product composition for enriching asthenic disease.

Example 2

The preparation process of the health-care product composition for enriching asthenic disease of the present example is as follows:

(1) according to parts by mass, 30 parts of dandelions, 10 parts of scallions, 10 parts of Chinese chives, 8 parts of mixtures of lemons with tangerines and oranges, 3 parts of jinhua fungus of tea seeds, 3 parts of pine pollens, 3 parts of mixtures of barbary wolfberry fruits with emblic and seabuckthorn fruits, and 3 parts of cinnamons are pulverized and mixed so as to obtain a solid mixture;

(2) 800 ml of purified water are added to the above-mentioned solid mixture; the mixture is subjected to leaching for 100 min after the temperature raising to 70°C, and filtered to obtain a leaching juice;

(3) the above-mentioned leaching juice is concentrated to obtain a concentrated liquid having a relative density of 1.20 under a pressure of a reverse osmotic pressure of 100 kg/cm$^2$;

(4) ethanol solution (75% by volume percentage content of ethanol) with an volume 3 times the volume of the concentrated liquid is added to the above-mentioned concentrated liquid to precipitate and remove impurities so as to obtain a refining solution;

(5) β-cyclodextrin having a volume of 0.6% of the concentrated liquid volume is added to the above-mentioned refining solution, stirred and mixed for embedding treatment so as to obtain an extracting solution;

(6) the above-mentioned extracting solution is lyophilized at -70°C so as to prepare a lyophilized powder of the health-care product composition for enriching asthenic disease;

(7) according to parts by mass, 5 parts of brown sugars are dried at 60°C for 12 hours, pulverized and passed through a 60 mesh sieve to obtain a treated brown sugar product; 0.1 parts of the table salts are dried at 105°C for 3 hours, cooled at room temperature and passed through a 16 mesh sieve to obtain a treated table salt product; the lyophilized powder, the treated brown sugar product, the treated table salt product, and 0.1 parts of essences are mixed at 12°C to obtain a solid beverage of the health-care product composition for enriching asthenic disease.

Example 3

The preparation process of the health-care product composition for enriching asthenic disease of the present example is as follows:

(1) according to parts by mass, 20 parts of dandelions, 10 parts of scallions, 80 parts of Chinese chives, 14 parts of mixtures of litchis and longans, 6 parts of mixtures of pine seeds with moringa seeds and seeds of *Lepidium meyenii Walp.,* and 3 parts of fennels are pulverizeed and mixed so as to obtain a solid mixture;

(2) 800 ml of purified water are added to the above-mentioned solid mixture; the mixture is subjected to leaching for 60 min after the temperature raising to 70°C, and filtered to obtain a leaching juice;

(3) the above-mentioned leaching juice is concentrated to obtain a concentrated liquid having a relative density of 1.20 under a pressure of a reverse osmotic pressure of 150 kg/cm$^2$;

(4) ethanol solution (75% by volume percentage content of ethanol) with an volume 3 times the volume of the concentrated liquid is added to the above-mentioned concentrated liquid to precipitate and remove impurities so as to obtain a refining solution;

(5) β-cyclodextrin having a volume of 1.5% of the concentrated liquid volume is added to the above-mentioned refining solution, stirred and mixed for embedding treatment so as to obtain an extracting solution;

(6) according to parts by mass, 3 parts of glucose and 0.01 parts of table salt are added to the above-mentioned extracting solution and the mixture is heated to 93°C for sterilization for 3 minutes so as to formulate and prepare a liquid beverage of the health-care product composition for enriching asthenic disease.

Example 4

The preparation process of the health-care product composition for enriching asthenic disease of the present example is as follows:

(1) according to parts by mass, 30 parts of dandelions, 30 parts of scallions, 10 parts of Chinese chives, 11 parts of mixtures of cherries with strawberries and tomatoes, 6 parts of jinhua fungus of tea seeds and 6 parts of pine pollens are pulverized and mixed so as to obtain a solid mixture;

(2) 800 ml of purified water are added to the above-mentioned solid mixture; the mixture is subjected to leaching for 120 min after the temperature raising to 75°C, and filtered to obtain a leaching juice;

(3) the above-mentioned leaching juice is concentrated to obtain a concentrated liquid having a relative density of 1.20 under a pressure of a reverse osmotic pressure of 80 kg/cm$^2$;

(4) 3 times of volume of ethanol solution (96% by volume percentage content of ethanol) are added to the above-mentioned concentrated liquid to precipitate and remove impurities so as to obtain a refining solution;

(5) 1.5% of β-cyclodextrin is added to the above-mentioned refining solution, stirred and mixed for embedding treatment so as to obtain an extracting solution;

(6) the above-mentioned extracting solution is lyophilized at -50°C so as to prepare a lyophilized powder of the health-care product composition for enriching asthenic disease;

(7) according to parts by mass, 4 parts of white granulated sugars are dried at 60°C for 12 hours, pulverized and passed through a 60 mesh sieve to obtain a treated white granulated sugar product; 0.05 parts of the table salts are dried at 105°C for 3 hours, cooled at room temperature and passed through a 16 mesh sieve to obtain a treated table salt product; the lyophilized powder, the treated white granulated sugar product, the treated table salt product, 0.5 parts of sodium bicarbonate, and 0.3 parts of the flavoring materials are mixed at 12°C to obtain a solid beverage of the health-care product composition for enriching asthenic disease.

Example 5

The preparation process of the health-care product composition for enriching asthenic disease of the present example is as follows:

(1) according to parts by mass, 40 parts of dandelions, 120 parts of scallion seeds, 10 parts of Chinese chives, 16 parts of mixtures of pineapples with coconuts and pitaya, 3 parts of mixtures of carthamus seeds with perilla fruits and okra seeds, and 3 parts of ground peppers are pulverizeed and mixed so as to obtain a solid mixture;

(2) 800 ml of purified water are added to the above-mentioned solid mixture; the mixture is subjected to leaching for 90 min after the temperature raising to 75°C, and filtered to obtain a leaching juice;

(3) the above-mentioned leaching juice is concentrated to obtain a concentrated liquid having a relative density of 1.20 under a pressure of a reverse osmotic pressure of 100 kg/cm$^2$;

(4) ethanol solution (75% by volume percentage content of ethanol) with an volume 3 times the volume of the concentrated liquid is added to the above-mentioned concentrated liquid to precipitate and remove impurities so as to obtain a refining solution;

(5) β-cyclodextrin having a volume of 3% of the concentrated liquid volume is added to the above-mentioned refining solution, stirred and mixed for embedding treatment so as to obtain an extracting solution;

(6) the above-mentioned extracting solution is lyophilized at -50°C so as to prepare a lyophilized powder of the health-care product composition for enriching asthenic disease;

(7) according to parts by mass, 3 parts of rock sugars are dried at 60°C for 12 hours, pulverized and passed through a 60 mesh sieve to obtain a treated rock sugar product; 0.2 parts of the table salts are dried at 105°C for 3 hours, cooled at room temperature and passed through a 16 mesh sieve to obtain a treated table salt product; the lyophilized powder, the treated rock sugar product, the treated table salt product, 0.5 parts of sodium bicarbonate, and 0.3 parts of the flavoring materials are mixed at 12°C to obtain a solid beverage of the health-care product composition for enriching asthenic disease.

Example 6

The preparation process of the health-care product composition for enriching asthenic disease of the present example is as follows:

(1) according to parts by mass, 40 parts of dandelions, 10 parts of stalks of the scallions, 100 parts of seeds of Chinese chives, 9 parts of mixtures of yacons and durians, 3 parts of mixtures of oats with tartarian buckwheats and tree peony seeds, and 6 parts of *Ligusticum chuanxiong Hort.* are pulverizeed and mixed so as to obtain a solid mixture;

(2) 800 ml of purified water are added to the above-mentioned solid mixture; the mixture is subjected to leaching for 90 min after the temperature raising to 75°C, and filtered to obtain a leaching juice;

(3) the above-mentioned leaching juice is concentrated to obtain a concentrated liquid having a relative density of 1.20 under a pressure of a reverse osmotic pressure of 100 kg/cm$^2$;

(4) ethanol solution (80% by volume percentage content of ethanol) with an volume 3 times the volume of the concentrated liquid is added to the above-mentioned concentrated liquid to precipitate and remove impurities so as to obtain a refining solution;

(5) porous starch having a volume of 1.5% of the concentrated liquid volume is added to the above-mentioned refining solution, stirred and mixed for embedding treatment so as to obtain an extracting solution;

(6) the above-mentioned extracting solution is lyophilized at -60°C so as to prepare a lyophilized powder of the health-care product composition for enriching asthenic disease;

(7) according to parts by mass, 3 parts of brown sugars are dried at 60°C for 12 hours, pulverized and passed through a 60 mesh sieve to obtain a treated brown sugar product; 0.3 parts of the table salts are dried at 105°C for 3 hours, cooled at room temperature and passed through a 16 mesh sieve to obtain a treated table salt product; the lyophilized powder, the treated brown sugar product, the treated table salt product, 0.5 parts of sodium bicarbonate, and 0.2 parts of the flavoring materials are mixed at 12°C to obtain a solid beverage of the health-care product composition for enriching asthenic disease.

Example 7

The preparation process of the health-care product composition for enriching asthenic disease of the present example is as follows:

(1) according to parts by mass, 10 parts of dandelions, 15 parts of onions, 25 parts of celeries, 8 parts of noni fruits, 6 parts of jinhua fungus of tea seeds, 6 parts of pine pollens, 6 parts of mixtures of mulberry fruits with passion fruits and hawthorn fruits, and 3 parts of pricklyash peels are pulverized and mixed so as to obtain a solid mixture;

(2) 800 ml of purified water are added to the above-mentioned solid mixture; the mixture is subjected to leaching for 120 min after the temperature raising to 80°C, and filtered to obtain a leaching juice;

(3) the above-mentioned leaching juice is concentrated to obtain a concentrated liquid having a relative density of 1.20 under a pressure of a reverse osmotic pressure of 100 kg/cm$^2$;

(4) ethanol solution (85% by volume percentage content of ethanol) with an volume 3 times the volume of the concentrated liquid is added to the above-mentioned concentrated liquid to precipitate and remove impurities so as to obtain a refining solution;

(5) porous starch having a volume of 5% of the concentrated liquid volume is added to the above-mentioned refining solution, stirred and mixed for embedding treatment so as to obtain an extracting solution;

(6) the above-mentioned extracting solution is lyophilized at -35°C so as to prepare a lyophilized powder of the health-care product composition for enriching asthenic disease;

(7) according to parts by mass, 4 parts of fructoses are dried at 60°C for 12 hours, pulverized and passed through a 60 mesh sieve to obtain a treated fructose product; 0.15 parts of the table salts are dried at 105°C for 3 hours, cooled at room temperature and passed through a 16 mesh sieve to obtain a treated table salt product; the lyophilized powder, the treated fructoses product, the treated table salt product, 0.3 parts of sodium bicarbonate, and 0.3 parts of the flavoring materials are mixed at 12°C to obtain a solid beverage of the health-care product composition for enriching asthenic disease.

Example 8

The preparation process of the health-care product composition for enriching asthenic disease of the present example is as follows:

(1) according to parts by mass, 5 parts of dandelions, 10 parts of garlics, 30 parts of stalks of Chinese chives, 6 parts of *Solanum mammosum L.*, 6 parts of jinhua fungus of tea seeds, 6 parts of pine pollens, 3 parts of mixtures of walnut seeds with bitter apricot seeds and peach seeds, and 6 parts of cloves are pulverized and mixed so as to obtain a solid mixture;

(2) 800 ml of purified water are added to the above-mentioned solid mixture; the mixture is subjected to leaching for 60 min after the temperature raising to 80°C, and filtered to obtain a leaching juice;

(3) the above-mentioned leaching juice is concentrated to obtain a concentrated liquid having a relative density of 1.20 under a pressure of a reverse osmotic pressure of 100 kg/cm$^2$;

(4) ethanol solution (96% by volume percentage content of ethanol) with an volume 3 times the volume of the concentrated liquid is added to the above-mentioned concentrated liquid to precipitate and remove impurities so as to obtain a refining solution;

(5) porous starch having a volume of 5% of the concentrated liquid volume is added to the above-mentioned refining solution, stirred and mixed for embedding treatment so as to obtain an extracting solution;

(6) the above-mentioned extracting solution is lyophilized at -35°C so as to prepare a lyophilized powder of the health-care product composition for enriching asthenic disease;

(7) according to parts by mass, 0.05 parts of egg white and sugars are dried at 60°C for 12 hours, pulverized and passed through a 60 mesh sieve to obtain a treated egg white and sugar product; 0.05 parts of the table salts are dried at 105°C for 3 hours, cooled at room temperature and passed through a 16 mesh sieve to obtain a treated table salt product; the lyophilized powder, the treated egg white and sugar product, the treated table salt product, 0.4 parts of sodium bicarbonate, and 0.2 parts of the flavoring materials are mixed at 12°C to obtain a solid beverage of the health-care product composition for enriching asthenic disease.

Example 9

The preparation process of the health-care product composition for enriching asthenic disease of the present example is as follows:

(1) according to parts by mass, 5 parts of dandelions, 25 parts of Chinese onions, 10 parts of corianders, 8 parts of durians, 6 parts of jinhua fungus of tea seeds, 6 parts of pine pollens, 3 parts of mixtures of natto with cacao beans and coffee beans, and 2 parts of star anis are pulverized and mixed so as to obtain a solid mixture;

(2) 800 ml of purified water are added to the above-mentioned solid mixture; the mixture is subjected to leaching for 60 min after the temperature raising to 80°C, and filtered to obtain a leaching juice;

(3) the above-mentioned leaching juice is concentrated to obtain a concentrated liquid having a relative density of 1.20 under a pressure of a reverse osmotic pressure of 100 kg/cm$^2$;

(4) ethanol solution (75% by volume percentage content of ethanol) with an volume 3 times the volume of the concentrated liquid is added to the above-mentioned concentrated liquid to precipitate and remove impurities so as to obtain a refining solution;

(5) porous starch having a volume of 6% of the concentrated liquid volume is added to the above-mentioned refining solution, stirred and mixed for embedding treatment so as to obtain an extracting solution;

(6) the above-mentioned extracting solution is lyophilized at -35°C so as to prepare a lyophilized powder of the health-care product composition for enriching asthenic disease;

(7) according to parts by mass, 0.01 parts of steviosides are dried at 60°C for 12 hours, pulverized and passed through a 60 mesh sieve to obtain a treated stevioside product; 0.02 parts of the table salts are dried at 105°C for 3 hours, cooled at room temperature and passed through a 16 mesh sieve to obtain a treated table salt product; the lyophilized powder, the treated stevioside product, the treated table salt product, 0.3 parts of sodium bicarbonate, and 0.3 parts of the flavoring materials are mixed at 12°C to obtain a solid beverage of the health-care product composition for enriching asthenic disease.

Test section:
the following clinical trials are carried out based on the examples and the traditional Chinese medicine: Shenrong Sanbian powder and Xidekang powder:

1. Information for experimental subject

all observation subjects are people in sub-health status and clinically diagnosed deficiency of the kidney, a total of 36 cases; all of the subjects are male, with the youngest 21 years old, the oldest 66 years old, and the average age of 44 years old.

People in sub-health status are divided into three groups, 12 in each group, named as groups 1 to 3 respectively; the SPSS statistical software is used to compare the ages and the scores of traditional Chinese medicine symptoms of 3 groups, with $P > 0.05$ showing no statistically significant difference, which indicates that 3 groups are comparable.

2. Selection criteria for experimental subjects

2.1 Case inclusion criteria

(1) people diagnosed clinically sub-health status;
(2) people aged 18 to 75 years old;
(3) people sign the informed consent form.

2.2 Case exclusion criteria

(1) people with a history of allergies;
(2) people accompanied by primary diseases such as liver disease, kidney disease, hematopoietic system disease, heart disease and cerebrovascular disease, and mental patients;
(3) people with abnormal liver functions;
(4) people with other organ lesions.

3. Experimental method

People in sub-health status of group 1 respectively take the solid powder obtained from the composition of the present invention prepared in example 1; people of group 2 and group 3 respectively take the traditional Chinese medicine Shenrong Sanbian powder and Xidekang powder prepared in comparative examples 1 and 2 (15 g each time, twice a day), and the physical response of the experimental subjects are checked and recorded.

4. Evaluation criteria for traditional Chinese medicine syndrome

Deficiency of the kidney, deficiency of essence, deficiency of qi and blood, insomnia, forgetfulness, lassitude in loin and legs, dizziness, tinnitus and other symptoms are divided into three levels: no symptom, mild symptom and severe symptom, with the corresponding scores of 0, 2 and 4 points; after 20 days of taking, observation subjects are scored.

$$\text{Efficacy index (n)} = [(\text{pre-treatment score} - \text{post-treatment score}) \div \text{pre-treatment score}] \times 100\%$$

Markedly effective: most of the above-mentioned symptoms in the syndrome disappear, with the effective rate: $n \geq 70\%$;
Effective: the above-mentioned symptoms in the syndrome basically disappear, with the effective rate: $30\% n \leq n < 70\%$;
Ineffective: the above-mentioned symptoms in the syndrome are improved to a certain extent, with the effective rate: $n < 30\%$;

$$\text{effective rate} = [\text{number of markedly effective cases} + \text{number of effective cases/total number of cases}] \times 100\%.$$

5. Experimental results:

Table 1 Clinical trial results

| Groups | Markedly effective (number of people) | Effective (number of people) | Ineffective (number of people) | Effective rate% |
|--------|---------------------------------------|------------------------------|--------------------------------|-----------------|
| 1 | 7 | 4 | 1 | 91.66 |
| 2 | 5 | 5 | 2 | 83.33 |
| 3 | 8 | 2 | 2 | 83.33 |

It can be seen from the results of table 1 that the health-care product composition for enriching asthenic disease prepared by the present invention has a good efficacy of enriching asthenic disease, tonifying kidney and strengthening yang in people in sub-health status of deficiency caused by kidney deficiency, with the effective rate reaching 91.66%.

6. Case feedback

Case 1: ZHOU, a 60-year-old male, for nearly a year has been weak and susceptible to diseases, had weak constitution, experienced general malaise, become tired easily; he has been tired after doing light work, got palpitation, felt weakness in his legs, usually accompanied by tinnitus; after he took 6 bottles of the liquid beverage of the present composition, the performance of the mental status was improved and fatigue was mitigated, which led to more comfortable status in the body; after he took a course of treatment for 20 days, the physical condition was improved, and the appearing symptoms disappeared; he was full of spirit and energy, and the physical strength returned to normal.
Case 2: ZHANG, a 24-year-old male, lives with his girlfriend for half a year and had unsatisfactory sexual life with weak erection, accompanied by premature ejaculation symptoms; he had history of masturbation before; after he took the powder of the present composition for one course of treatment, sexual function was recovered, and sexual life with his girlfriend lasted for 40 minutes, with strong sexual requirements.
Case 3: ZHU, a 29-year-old male, had symptoms of hypaphrodisia and frequent nocturia, accompanied with fatigue and weakness; he took the powder of the present composition; he had sexual impulse immediately after taking 1 bottle, and after taking 5 to 6 bottles continuously, he had good erectile strength, significantly-reduced nocturia, and obviously improved ability to think, which lasted for one week; his exercise response was more sensitive and quicker than before, and he did not feel tired when working at night; especially, he had a frequent sexual life and felt no fatigue after sexual intercourse.

In summary, the above-mentioned health-care product composition for enriching asthenic disease has at least the following beneficial effects:

(1) the above-mentioned health-care product composition for enriching asthenic disease contains no sex hormones, and has no obvious toxic and side effects because the raw materials required for preparing the above-mentioned health-care product composition for enriching asthenic disease are vegetables, fruits, edible seeds, etc. which are commonly used in daily life;

(2) the above-mentioned health-care product composition for enriching asthenic disease has been confirmed by clinical trials to has good effects of enriching asthenic disease, tonifying kidney and strengthening yang.

The technical features of the above-described examples may be combined arbitrarily. For the purpose of simplicity in description, all the possible combinations of the technical features in the above-described examples are not described. However, as long as there is no contradiction among the combinations of these technical features, they shall all fall within the scope of the specification.

The above-mentioned examples merely represent several examples of the present invention, giving specifics and details thereof, but should not be understood as limiting the scope of the present patent of invention thereby. It should be noted that a person of ordinary skill in the art could also make several alterations and improvements without departing from the spirit of the present invention and these would all fall within the scope of protection of the present invention. Therefore, the scope of protection of the present patent of invention shall be in accordance with the appended claims.

**Claims**

1. A health-care product composition for enriching asthenic disease, **characterized in that** according to parts by mass, the raw materials for preparing the health-care product composition for enriching asthenic disease comprise:

5 to 40 parts of dandelions, 10 to 120 parts of bulbous vegetables, 10 to 100 parts of condiment leafy vegetables, and 8 to 16 parts of fruits;
wherein the bulbous vegetables are selected from at least one of scallion, onion and garlic; the condiment leafy vegetables are selected from at least one of Chinese chive, celery and coriander; the fruits are selected from at least one of grape, blueberry, blackcurrant, lemon, tangerine, orange, litchi, cherry, longan aril, strawberry, pineapple, coconut, pitaya, yacon, durian, noni fruit, *Solanum mammosum L.* and tomato.

2. The health-care product composition for enriching asthenic disease according to claim 1, **characterized in that** according to parts by mass, the raw materials for preparing the health-care product composition for enriching asthenic disease further comprise: 0 to 6 parts of edible seeds, which are selected from at least one of barbary wolfberry fruit, emblic, seabuckthorn fruit, oat, tartarian buckwheat, natto, mulberry fruit, walnut seed, sesame, tree peony seed, passion fruit, bitter apricot seed, peach seed, carthamus seed, perilla fruit, cushaw seed, bitter gourd seed, pine seed, moringa seed, seed of *Lepidium meyenii Walp.,* okra seed, hawthorn fruit, cacao bean and coffee bean.

3. The health-care product composition for enriching asthenic disease according to claim 1, **characterized in that** according to parts by mass, the raw materials for preparing the health-care product composition for enriching asthenic disease further comprise 0 to 6 parts of jinhua fungus of tea seeds and 0 to 6 parts of pine pollens.

4. The health-care product composition for enriching asthenic disease according to claim 1, **characterized in that** according to parts by mass, the raw materials for preparing the health-care product composition for enriching asthenic disease further comprise: 0 to 6 parts of condiment materials, which are selected from at least one of ginger, cinnamon, fennel, star anise, pepper fruit, *Ligusticum chuanxiong Hort.,* pricklyash peel and clove.

5. Use of the health-care product composition for enriching asthenic disease according to any one of claims 1 to 4 for the preparation of a health-care medicine, a health-care beverage or chocolate.

6. A method for preparing the health-care product composition for enriching asthenic disease according to any one of claims 1 to 4, **characterized in that** the method comprises the following steps: the raw materials are pulverized, mixed, and then subjected to leaching treatment in purified water at 70°C to 80°C so as to obtain a leaching juice.

7. The method for preparing the health-care product composition for enriching asthenic disease according to claim 6, **characterized in that** after the step of the leaching treatment, the method further comprises the following step:

the leaching juice is concentrated to obtain a concentrated liquid having a relative density of 1.18 to 1.20; and an embedding medium having a volume of 0.3% to 6% of the concentrated liquid volume is added for embedding treatment so as to obtain an extracting solution.

8. The method for preparing the health-care product composition for enriching asthenic disease according to claim 7, **characterized in that** when concentration is performed, the reverse osmotic pressure is 30 kg/cm$^2$ to 150 kg/cm$^2$, and the embedding medium is $\beta$-cyclodextrin or porous starch.

9. The method for preparing the health-care product composition for enriching asthenic disease according to claim 7, **characterized in that** after the step of concentration treatment and before the step of embedding treatment, the method further comprises the following steps: ethanol solution with an volume 3 times the volume of the concentrated liquid is added to precipitate and remove the impurities so as to obtain a refining solution, wherein the volume percentage content of ethanol in the ethanol solution is higher than 70%.

10. The method for preparing the health-care product composition for enriching asthenic disease according to claim 7, **characterized in that** after the step of embedding treatment, the method further comprises the following step: the extracting solution is lyophilized at a temperature of -70°C to -35°C so as to prepare a lyophilized powder.

| Pulverizing and mixing the raw materials to obtain a solid mixture. | S110 |

| Adding purified water to the solid mixture for leaching treatment, and filtering to obtain a leaching juice. | S120 |

| Concentrating the leaching juice to obtain a concentrated liquid. | S130 |

| Refining the concentrated liquid by adding ethanol so as to obtain a refining solution. | S140 |

| Adding an embedding medium for embedding treatment so as to obtain an extracting solution. | S150 |

| Lyophilizing the extracting solution to obtain a lyophilized powder of the health-care product composition for enriching asthenic disease. | S160 |

| Adding adjuvant materials to the above-mentioned leaching juice or lyophilized powder; mixing and formulating to prepare a liquid beverage or a solid beverage. | S170 |

*Fig. 1*

## INTERNATIONAL SEARCH REPORT

| International application No. |
|---|
| **PCT/CN2019/110142** |

**A. CLASSIFICATION OF SUBJECT MATTER**

A23L 19/00(2016.01)i; A23L 33/00(2016.01)i

According to International Patent Classification (IPC) or to both national classification and IPC

**B. FIELDS SEARCHED**

Minimum documentation searched (classification system followed by classification symbols)

A23L

Documentation searched other than minimum documentation to the extent that such documents are included in the fields searched

Electronic data base consulted during the international search (name of data base and, where practicable, search terms used)

TWABS; DWPI; MOABS; TWMED; VEN; HKABS; CNABS; CNTXT; WOTXT; TWTXT; USTXT; EPTXT; CNKI; Elsevier Science; 百度学术; BAIDU: 蒲公英, 葱, 洋葱, 蒜, 韭菜, 芹菜, 芫荽, 香菜, 葡萄, 蓝莓, 黑加仑, 柠檬, 橘子, 橙子, 荔枝, 樱桃, 龙眼肉, 草莓, 菠萝, 椰子, 火龙果, 雪莲果, 榴莲, 诺丽果, 五指果, 西红柿, 枸杞, 余甘子, 沙棘, 燕麦, 苦荞, 纳豆, 桑椹, 核桃, 芝麻, 百香果, 杏仁, 桃仁, 紫苏, 南瓜, 苦瓜, 玛卡, 黄秋葵, 山楂, 可可, 咖啡, 茶籽金花菌, 松花粉, 姜, 肉桂, 八角, 茴香, Taraxacum mongolicum, shallot, onion, garlic, leek, celery, coriander, grape, blueberry, blackcurrant, lemon, orange, litchi, cherry, strawberry, pineapple, coconut, pitaya, tomato, mulberry, walnut, perilla, pumpkin, balsam pear, Maka, okra, hawthorn, cocoa, coffee, camellia, pine pollen, ginger, cinnamon

**C. DOCUMENTS CONSIDERED TO BE RELEVANT**

| Category* | Citation of document, with indication, where appropriate, of the relevant passages | Relevant to claim No. |
|---|---|---|
| X | CN 103622002 A (ZHEJIANG JINSHANMEI BIOTECHNOLOGY CO., LTD.) 12 March 2014 (2014-03-12)<br>claim 5 and description, paragraph 5 | 1-10 |
| A | CN 105614718 A (SHAOXING UNIVERSITY) 01 June 2016 (2016-06-01)<br>description, paragraphs 6-49 | 1-10 |
| A | CN 104096015 A (XIE, Youzhu) 15 October 2014 (2014-10-15)<br>entire document | 1-10 |
| A | CN 101467623 A (LIU, Yonghong) 01 July 2009 (2009-07-01)<br>claims 1-3 | 1-10 |
| A | CN 108308614 A (YANG, Fuwen) 24 July 2018 (2018-07-24)<br>claims 1 and 2 | 1-10 |

☐ Further documents are listed in the continuation of Box C.　　☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **23 December 2019** | **06 January 2020** |

| Name and mailing address of the ISA/CN | Authorized officer |
|---|---|
| **China National Intellectual Property Administration (ISA/CN)**<br>**No. 6, Xitucheng Road, Jimenqiao Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**
Information on patent family members

International application No.

**PCT/CN2019/110142**

| Patent document cited in search report | | | Publication date (day/month/year) | Patent family member(s) | Publication date (day/month/year) |
|---|---|---|---|---|---|
| CN | 103622002 | A | 12 March 2014 | None | |
| CN | 105614718 | A | 01 June 2016 | None | |
| CN | 104096015 | A | 15 October 2014 | None | |
| CN | 101467623 | A | 01 July 2009 | None | |
| CN | 108308614 | A | 24 July 2018 | None | |

Form PCT/ISA/210 (patent family annex) (January 2015)

**REFERENCES CITED IN THE DESCRIPTION**

**Patent documents cited in the description**

- HK 18112945